# EUROPEAN PATENT APPLICATION

(11) **EP 4 613 921 A1**
(43) Date of publication of application: **10.09.2025**
(21) Application number: 24162304.0
(22) Date of filing: 08.03.2024
(51) Int. Cl.: D03D 11/00, D03D 19/00, D03D 13/00

(54) **WOVEN MULTILAYER GAUZE**

(71) Applicant: Calik Denim Tekstil San. Ve Tic. A.S., 34169 Istanbul (TR)
(72) Inventor: BABAARSLAN, Osman, 34169 Istambul (TR); KAYA, Kemal, 34169 Istambul (TR); KORKMAZ, Ahmet, 34169 Istambul (TR)
(74) Representative: Kaya, Erdem

(57) **Abstract**

The invention relates to a woven multilayer gauze (21), comprising at least one multilayer section (23) with at least three individual layers, wherein the at least one multilayer section (23) is bordered from at least two sides by connecting sections (22, 28) in which yarns from each individual layer merge into a common layer.

## Description

The present invention relates to a woven multilayer gauze.

Gauzes (also known as gauze bandages) are used for wound dressings. To reduce the risk of infections by yarns adhering on wounds, gauzes are designed to avoid yarns to fall out of the gauze. For this purpose, gauzes are traditionally woven from individual layers having the length and width of the desired bandage. This allows to use a continuous weft yarn which substantially avoids open yarn ends, and thereby reduces the risk that yarns fall out of the gauze.

However, this traditional method is expensive, as gauze bandages usually have a width between 5 cm and 20 cm, thereby requiring specific weaving machines being designed for each width required. Further, due to the small width of those bandages, a high number of weft insertions are required per cm² of gauze bandage, making the process slow and expensive (as a weaving machine can only conduct a maximum number of weft insertions in a minute).

To solve this problem, JP 2005/021307 A suggests producing gauze bandages in two steps, namely to produce in a first step a woven fabric with relatively large width and to cut off in a second step gauze bandages from that woven fabric by cutting the woven fabric in warp direction into stripes. To avoid falling out of yarns in the cutting edge, JP 2005/021307 A suggests providing the cutting edge with increased warp density.

However, even the method of JP 2005/021307 A has turned out to not be fast enough and still too expensive. While the idea of increasing the warp density in the cutting areas indeed turned out to reduce the risk of yarns falling out of the fabric, this approach comes with a high material consumption, in particular of warp yarns in the cutting area.

It is an object of the present invention to overcome the drawbacks of the prior art, in particular to provide a woven gauze that can be produced cheaper, in particular faster and with lower material consumption, in particular without increasing the risk of yarns falling out of the bandage.

The object is solved by the independent claims. One aspect of the invention provides a woven multilayer gauze, comprising at least one multilayer section with at least three individual layers, wherein the at least one multilayer section is bordered from at least two sides by connecting sections in which yarns from each individual layer merge into a common layer.

The inventors have found that the inventive idea of unifying (merging) yarns from each individual layer of the multilayer section into common layers bordering the multilayer section allows to provide a cutting section of increased yarn density (to reduce the risk of yarns falling out) without having to use any additional yarns in this area, thereby keeping costs for the woven gauze low. For example, compared to the prior art where the warp density has to be increased in the cutting area, the present invention allows to keep the warp density in the cutting area constant or even to reduce it compared to the multilayer section, because
1.) the weft yarns merging from the multilayer section into the common layer already increase the yarn density in the common layer, and
2.) the warp density in the common layer can be used to hold the weft yarns of multiple layers together.

In other words, regarding point 2.), the warp density can (for example in the case of a multilayer section with four individual layers) be chosen to be double as high as the warp density in one individual layer, thereby reducing the risk of yarns falling out, while the overall warp density of the common layer is lower than the warp density of the multilayer section. Thus, even in embodiments in which the warp density in the common layer is larger than the warp density of one single layer (which is not necessary for the present invention in view of the increased density of the weft yarns merging into the common layer), the warp density in the common layer can be significantly reduced compared to the prior art. The inventors have found that this effect becomes pronounced with at least three individual layers in the multilayer section and becomes particularly strong with at least four, five or six individual layers in the multilayer section. Therefore, the multilayer section preferably comprises at least four, five, six, seven or eight individual layers, particularly preferred between three and ten individual layers, even more preferred between four and ten, five and ten, six and ten, seven and ten, or eight and ten individual layers.

A woven multilayer gauze within the meaning of if the present invention is in particular to be understood as a multilayer gauze where the at least one multilayer section and the connecting sections are woven in one step, in particular on a common weaving machine. As will be described in detail below, the woven multilayer gauze can comprise a plurality of multilayer sections and connecting sections. Preferably, a woven multilayer gauze within the meaning of this invention is a multilayer gauze in which all of the multilayer sections and connecting sections are woven in one step, in particular in one weaving machine/loom.

A multilayer gauze within the meaning of this invention is preferably a gauze having at least three individual layers stacked above each other. In this regard, the stacked shall not have the meaning of a process step of laying individual layers above each other. Rather, stacked shall only mean that the individual layers extend in vertical direction (the direction extending orthogonal to a plain defined by the warp yarn direction and weft yarn direction) above each other. Thereby, the three individual layers are particularly woven in a single step, in particular on a common weaving loom.

A gauze within the meaning of the present invention shall in particular be understood as a fabric being suitable to be used for wound dressings. For this purpose, a gauze within the meaning of this invention preferably comprises cotton, particularly preferably consists to at least 50%, 60%, 70%, 80%, 90%, 95%, 99% or 100% of cotton. Unless otherwise specified, precent values given in this disclosure relate to weight percent (wt.%). Additionally, or alternatively, a gauze within the meaning of this invention is bleached and/or treated with an anti-microbial treatment, in particular as described in detail below. Particularly preferred, to make the gauze suitable to be used for wound dressings, it comprises, in particular consists of, cotton, is bleached and treated by an anti-microbial treatment. Additionally or alternatively, a gauze within the meaning of this invention is free of any dye stuff, in particular is undyed/uncolored, to avoid infections in the case of fibers adhering to the wound.

Additionally or alternatively, the yarn count of the warp yarns and/or of the weft yarns of the gauze within the meaning of this invention is between 148 and 895 dtex (6/40 Ne), preferably between 197 and 590 dtex (10/30 Ne), even more preferably between 236 and 393 dtex (50/25 Ne), most preferably between 268 and 328 dtex (80/22 Ne).

The fabric weight of a gauze within the meaning of this invention is preferably between 40 and 100 g/m², more preferably between 45 and 80 g/m², most preferably between 55 and 70 g/m², per layer of the multilayer section. For instance, the fabric weight can be 63 g/cm² for each layer of the multilayer section. In other words, in case of a gauze with a multilayer section having four individual layers, the fabric weight is preferably 252 g/cm².

Additionally or alternatively, the warp density of an individual layer of the multilayer section of a gauze within the meaning of this invention is between 3 and 50 warps/cm, preferably between 4 and 12 warps/cm, more preferably between 5 and 10 warps/cm, most preferably between 6 and 8 warps/cm, for instance about 7 warps/cm. Additionally or alternatively, the weft density of each layer of the multilayer section of a gauze within the meaning of this invention is between 5 and 20 wefts/cm, preferably between 6 and 18 wefts/cm, more preferably between 7 and 15 wefts/cm, most preferably between 8 and 12 wefts/cm.

Particularly preferred, a gauze within the meaning of this invention is a woven fabric comprising at least 80%, preferably 100%, cotton, as well as a warp yarn and weft yarn count between 197 and 590 dtex, preferably between 268 and 328 dtex, a warp density between 4 and 12 warps/cm, preferably between 6 and 8 warps/cm, in each individual layer of the multilayer section, a weft density between 6 and 80 wefts/cm, preferably between 8 and 12 wefts/cm, in each individual layer of the multilayer section, and preferably a fabric weight between 30 and 100 g/m², preferably between 45 and 70 g/m², in each individual layer of the multilayer section. Particularly preferred, the previously described preferred gauze is in addition bleached and/or treated with an anti-microbial treatment, particularly as described below, and preferably free of any dye stuff, particularly is undyed/uncolored.

In a particularly preferred embodiment, the woven multilayer gauze consists of cotton, has a warp yarn count of 295 dtex (20 Ne), a weft yarn count of 295 dtex (20 Ne), wherein each individual layer of the multilayer section has a warp density of 7 warps/cm, a weft density of 10 wefts/cm and a fabric weight of 63 g/m², wherein the gauze is preferably bleached and/or treated by an anti-microbial treatment, in particular as described below, and preferably undyed.

Preferably, the at least three individual layers of the multilayer section are stacked, in other words extend in vertical direction above each other. Preferably, the multilayer section comprises between three and ten individual layers, particularly preferred between four and ten individual layers. An individual layer within the meaning of this invention is in particular a layer extending along a certain warp length and weft length completely individual from layers stacked above and/or below that layer. Preferably, an individual layer extends along the complete extension of the multilayer section in warp direction and weft direction independent from other layers extending above or below that individual layer. Independent in this regard particularly means that neither warp yarns nor weft yarns of other individual layers in the same weft yarn section are interwoven with the warp yarns or weft yarns of that independent individual layer. In other words, each individual layer is preferably exclusively woven from a set of warp yarns and weft yarns of that individual layer, without interfering with warp yarns or weft yarns of other individual layers. Preferably, each individual layer extends along at least 3 cm, preferably at least 5 cm, 8 cm or 10 cm, in warp and/or weft direction individually. Particularly preferred, each individual layer extends independently along the entire extension of the multilayer section in warp direction and weft direction. Preferably, the multilayer section extends along at least 30 cm, preferably at least 50 cm, more preferably at least 100 cm, most preferably at least 150 cm, in one direction selected from the warp direction and the weft direction, preferably in the weft direction, and along at least 2 cm, preferably at least 3 cm, more preferably at least 5 cm, 8 cm or 10 cm, in the other direction selected from the weft direction and warp direction, preferably in warp direction. Particularly preferred, the multilayer section extends in weft direction along between 30 cm and 400 cm, more preferably between 50 cm and 300 cm, even more preferably between 100 cm and 250 cm, most preferably between 140 cm and 200 cm or between 190 cm and 200 cm. Additionally or alternatively, the multilayer section preferably extends in warp direction along between 2 cm and 30 cm, more preferably between 3 cm and 20 cm, most preferably between 5 cm and 10 cm. Particularly preferred, the multilayer section extends in warp and weft direction in the shape of a strip, particularly a strip having a length being at least 50 %, 100 %, 200 %, 300 % or 500 %, 1.000 % or 2.000 % larger than the width, preferably the length of the strip is in weft direction whereas the width of the strip is in warp direction. The inventors have found that, in particular by designing the multilayer section to extend along its length in weft direction, the woven multilayer gauze can be wrapped in warp direction around a roll, wherein unwrapping the multilayer section from the roll by a few centimeters allows to cut off a gauze bandage having substantially the length of the roll. As it is known in the art, woven fabrics can be produced on large rolls, in particular with lengths in weft direction of more than 1 m or 2 m. This allows to sell woven multilayer gauzes wrapped on a roll to hospitals, where they can, on demand, cut off gauze bandages of more than 1 m, 2 m or 4 m by unwrapping only a few centimeters of the rolled fabric from the roll.

A common layer of a connecting section within the meaning of this invention is in particular to be understood as a layer comprising connecting yarns extending parallel to the multilayer section, wherein the connecting yarns are interwoven with yarns of each individual layer merging from the multilayer section into the common layer. In other words, the yarns merging from the multilayer section into the common layer are preferably interwoven into a common single layer formed from the connecting yarns and the yarns merging from the multilayer section into the common layer. For example, when the connecting section borders the multilayer section in weft direction, the connecting yarns extend in warp direction and are interwoven in a single common layer with at least one, preferably all, weft yarn/s of each layer of the multilayer section. In yet other words, the common layer preferably unifies the yarns of the individual layers of the multilayer section, which extend into the connecting section, into a single layer, in particular by interweaving them with the connecting yarns of the common layer in the connecting section.

Preferably, the connecting section, in particular the common layer, extends parallel to the edge of the multilayer section bordered by the connecting section along the same length as the length the of the multilayer section in weft direction. Additionally or alternatively, the connecting section, particularly the common layer, extends between 1 cm and 10 cm, preferably between 2 cm and 8 cm or between 2 cm and 5 cm, in the direction orthogonal to the edge of the multilayer section bordered by the connecting section.

According to an embodiment, the at least one multilayer section is bordered in weft direction on both sides by weft connecting sections in which the weft yarns from each layer of the multilayer section merge into a common layer. In particular, the common layer of the weft connecting section comprises warp yarns as connecting yarns, which can also be referred to as warp connecting yarns. The warp connecting yarns are in particular interwoven with weft yarns, in particular all weft yarns, of each individual layer, in particular wherein the common layer is a single layer. In one embodiment, the woven multilayer gauze can comprise two weft connecting sections bordering the woven multilayer gauze in weft direction from both sides. In a particular embodiment, the weft connecting sections extend in warp direction along the complete length of the multilayer gauze. The section in between these two weft connecting sections of the multilayer gauze can be realized as one continuous multilayer section or as a plurality of multilayer sections arranged behind each other in warp and/or weft direction being spaced from each other by connection section, in particular warp connection sections and/or weft connection sections. As described previously in detail, a particular preferred embodiment provides a plurality of multilayer sections spaced behind each other in warp direction and preferably extending in weft direction along the entirety of the woven gauze between the two weft connecting sections.

In a preferred embodiment of a woven gauze with weft connecting sections, the warp yarn density in the weft connecting sections is maximally as high, in particular lower than, the warp density of the multilayer section. Preferably, the warp density in the weft connecting sections is between 100% and 80%, more preferably between 30% and 70%, most preferably between 40% and 60%, of the warp density of the multilayer section. For instance, the inventors have found that using half the warp density of the multilayer section in the weft connecting section allows binding the weft yarns merging from the multilayer section to the common layer tight enough to reduce the risk of fraying out or falling off of yarns. For instance, in case of a woven gauze having a multilayer section with four individual layers, each layer can have a warp density of 7 warps/cm, so that the overall warp density of the multilayer section is 28 warps/cm, wherein the warp density in the weft connecting section is 14 warps/cm. In general, it is preferred to produce each layer of the multilayer section with a warp density between 3 and 15 warps/cm, preferably 4-12 warps/cm, more preferably 5-10 warps/cm, most preferably 6-8 warps/cm, and the weft connecting section with a warp density maximally as high as the overall warp density of the multilayer section, preferably between 20 and 80%, more preferably between 30 and 70%, most preferably between 40 and 60% of the overall warp density of the multilayer section.

Preferably, at least one, at least two, at least three or at least four, preferably each, of the individual layers of the multilayer section have a plain weave or a twill weave, preferably a plain weave. Additionally or alternatively, at least one, preferably at least 20%, 30%, 40%, 50%, 60%, 70% or 80%, most preferably each, weft yarn in the common layer of at least one, preferably each, of the weft connection sections extends over a single warp yarn and subsequently under a single warp yarn, i.e. a 1/1 weave, wherein preferably the 1/1 weave is repeated along the entire width of the weft connection section in weft direction.

According to a further embodiment, which can be combined with the previous and subsequent embodiments, at least one, preferably each, warp yarn in the common layer of at least one, preferably each, of the weft connection sections extends over as much weft yarns as the number of individual layers in the multilayer section and subsequently under exactly as much weft yarns as the number of individual layers of the multilayer section. For instance, in case of a multilayer section comprising four individual layers, the warp yarns in the weft connection section preferably extend over four weft yarns and subsequently under four weft yarns. Preferably, the over portions of adjacent warp yarns in the weft connection section are arranged in a diagonal pattern.

According to a further embodiment, which can be combined with the previous and subsequent embodiments, the at least one multilayer section is bordered in warp direction on both sides by warp connecting sections in which the warp yarns from each layer of the multilayer section emerge into a common layer. In the common layer of the warp connecting sections, the connecting yarns are formed by weft yarns which are preferably woven into one single layer with warp yarns, in particular at least 10%, 20%, 30%, 40%, 50%, 60%, 70%, 80%, 90% or 100% of the warp yarns, of at least one, preferably of at least two, at least three, or each of, the individual layers of the multilayer section. Preferably, the warp connecting sections extend in warp direction along between 1-10 cm, preferably between 2-8 cm or 2-5 cm. In weft direction, the warp connecting section preferably extends along the entire extension of the multilayer section, preferably about 30-400 cm, more preferably between 50 cm and 300 cm, even more preferably between 100 cm and 250 cm, most preferably between 140 cm and 200 cm.

Particularly preferred, the multilayer section is bordered in warp direction on both sides by warp connecting sections and in weft direction on both sides by weft connecting sections.

In a preferred embodiment, the weft density in the warp connecting section is maximally as high as, in particular lower than, the weft density of the multilayer section, preferably between 20% and 80%, more preferably between 40% and 70%, most preferably between 40% and 60%, of the weft density ind the multilayer section. In particular, the embodiments and/or preferred ranges described with respect to the warp density in the weft connecting sections apply mutatis mutandis to the weft density in the warp connecting sections.

According to a further embodiment, which can be combined with the previous embodiments and vice versa, at least one, preferably each of, the warp connection sections comprises for each individual layer of the multilayer section at least one weft yarn extending over all warp yarns merging from that individual layer into the warp connection section and under all warp yarns merging from the other individual layers into the warp connection section. For instance, in the case of a multilayer section with three individual layers, among them a top layer, a middle layer and a bottom layer, the warp connection section comprises at least one weft yarn extending over all warp yarns merging from the top layer into the warp connection section and under all warp yarns merging from the middle layer and the bottom layer in to the warp connection section. In the same way, at least a second weft yarn is provided merging over all warp yarns merging from the middle layer into the warp connection section and under all warp yarns merging from the top and the bottom layers into the warp connection section. Further, at least a third weft yarn is provided in the warp connection section extending over all warp yarns merging from the bottom layer into the warp connection section and under all warp yarns merging from the top and middle layers into the warp connection section. According to another embodiment of the invention, which can be combined with the previous and subsequent embodiments, at least one, preferably each, warp yarn in the common layer of at least one, preferably each, of the warp connection sections extends under a single weft yarn and subsequently over exactly as much weft yarns as the number of individual layers of the multilayer section minus one. For instance, in the case of a multilayer section comprises three layers, at least one, preferably each, warp yarn in the common layer of at least one, preferably each, of the warp connection sections extends under a single weft yarn and subsequently over exactly over two weft yarns and thus has a 1/2 weave structure. In case of a multilayer section with four layers, the weave structure is preferably 1/3. In case of a multilayer section with five layers, the weave structure is preferably 1/4, and so on, up to embodiments in which the multilayer section comprises ten layers, where the preferred weave structure is 1/9. Preferably, the previously described weave structure is repeated over the entire length of the warp connecting section in warp direction and preferably merges into a plain weave in the individual layers of the multilayer section. Preferably, the under portions of the weft yarns in the warp connection section build a diagonal pattern.

In a further embodiment, which can be combined with the previous and subsequent embodiments, the woven gauze comprises at least two multilayer sections with at least three individual layers, wherein each multilayer section is bordered from at least two sides by connecting sections in which yarns from each individual layer merge into a common layer, wherein the two multilayer sections are arranged behind each other in warp or weft direction and are spaced from each other by one of the connection sections. Preferably, the at least two multilayer sections are arranged behind each other in warp direction and spaced from each other by warp connection sections, in particular as described above, and bordered in weft direction by weft connection sections, in particular as described above. Preferably, instead of at least two multilayer sections, at least three, four, five, six, eight or ten multilayer sections are arranged behind each other in warp direction.

In a further embodiment, which can be combined with the previous and subsequent embodiments, the woven gauze comprises at least four multilayer sections with at least three individual layers, wherein each multilayer section is bordered from at least two sides by connecting sections in which yarns from each individual layer merge into a common layer, wherein at least two multilayer sections are arranged behind each other in warp direction and are spaced from each other by one of the connection sections, in particular by a warp connection section, and wherein at least two multilayer sections are arranged behind each other in weft direction and are spaced from each other by one of the connection sections, in particular by a weft connection section as described above.

According to a further embodiment, which can be combined with the previous and subsequent embodiments, the woven gauze is wrapped in circumferential direction around the longitudinal axis of a roll, preferably wherein the woven gauze has a length in warp direction of at least 5 m, preferably at least 10 m, 20 m, 30 m, 50 m or 100 m, and a width in width direction of at least 0.5 m, preferably at least 1 m or at least 2 m. Particularly preferred, the woven gauze has a length in weft direction between 0.5 m and 4 m, preferably between 1 m and 3 m , more preferably between 1.5 m and 2 m, and a length in warp direction of between 5 m and 1,000 m, more preferably between 10 m and 500 m, even more preferably between 20 m and 200 m.

In a particularly preferred embodiment, the at least one multilayer section is bordered in circumferential direction from both sides by two connecting sections in which yarns, preferably warp yarns, from each individual layer merge into a common layer. Preferably, the two connecting sections are warp connecting sections, in particular as described above. Particularly preferred, the multilayer section extends in warp direction along between 3 cm and 50 cm, more preferably between 4 cm and 20 cm, most preferably between 5 cm and 10 cm.

Thereby, particularly as described above, a gauze bandage with the length of the woven gauze in longitudinal direction of the roll, for instance between 0.5 m and 4 m, can be unwrapped from the roll by unwrapping only the width of the gauze bandage from the roll.

In a further embodiment, which can be combined with the previous and subsequent embodiments, the at least one multilayer section is bordered along the longitudinal direction from both sides by two connecting sections, in which yarns, preferably weft yarns, from each individual layer merge into a common layer. Preferably, the connecting sections are weft connecting sections, in particular as described above. Particularly preferred, the weft connecting sections extend in circumferential direction along the entire length of the woven gauze and thereby borders the at least one or the plurality of multilayer sections located in between along the entire length of the woven multilayer gauze, in particular in warp direction.

According to a further embodiment, the woven gauze according to the invention or according to any of the previous and subsequent embodiments, is enclosed by a cover, in particular a plastic cover, more particularly a removable plastic cover. This embodiment is preferably combined with one or more of the previously described embodiments, where the woven gauze is wrapped in circumferential direction around the longitudinal axis of the roll. In such embodiment, preferably the entire roll including the woven gauze wrapped thereon is enclosed, in particular in an airtight manner, by a cover. This cover protects the woven gauze from pollution or microbial contamination during transportation, for instance from a weaving plant to a hospital.

The invention also relates to a method of producing a gauze bandage comprising the steps of
1.) producing a woven multilayer gauze comprising at least two multilayer sections with at least three individual layers, wherein each multilayer section is bordered from at least two sides by connecting sections in which yarns from each individual layer merge into a common layer, wherein the two multilayer sections are arranged behind each other in warp or weft direction and are spaced from each other by one of the connection sections ; and
2.) cutting at least one gauze bandage from the woven gauze by separating the multilayer sections from each other by cutting through at least one connecting section spacing both multilayer sections from each other.

In preferred embodiments of the invention, the woven gauze is treated with an anti-microbial treatment. Preferred embodiments of the invention relate to treating a gauze, or warp yarns and weft yarns of the invention with an "anti-microbial" treatment. An anti-microbial treatment in the context of the invention is a composition that comprises an anti-microbial agent. In particular preferred embodiments, the anti-microbial treatment comprises a wetting agent, an anti-microbial agent, and a cross-linking agent.

In preferred embodiments, the anti-microbial agent is an antiseptic, antibiotic, antiviral, antifungal and/or antiparasitic agent. In preferred embodiments, the anti-microbial agent comprises cannabidiol (CBD), preferably microencapsulated cannabidiol. Cannabidiol is also known as 2-[(1R,6R)-6-lsopropenyl-3-methylcyclohex-2-en-1-yl]-5-pentylbenzene-1,3-diol. It is known to have anti-inflammatory and antioxidant properties. Furthermore, it exhibits anti-microbial properties, for example against Gram-positive bacteria.

In preferred embodiments, the anti-microbial agent is microencapsulated, therefore in a microencapsulated form. By providing the anti-microbial agent in a microencapsulated form, the anti-microbial agent is released over time. This prolongs the duration of the anti-microbial effect of the anti-microbial treatment.

The term "microencapsulation" preferably refers to a process, in which a compound (such the anti-microbial agent of the invention) is enclosed into a "microcapsule". Thus, in preferred embodiments, the anti-microbial agent is enclosed by a microcapsule. In preferred embodiments, the microcapsule comprises a polymeric hull. Preferably, the polymeric hull of the microcapsule has a diameter of 1 to 300 µm, preferably of 2 to 100 µm more preferably of 2 to 50 µm. Preferably, the size of the microcapsule is determined by scanning electron microscopy using an acceleration voltage of 5 kV. Preferably, the polymeric hull is impervious to the composition in the core of the microcapsule. Exemplary polymeric shell materials include gelatine, polyurethane, polyolefin, polyamide, polyester, polysaccharide, silicone resins, chitosan and epoxy resins. Polymeric microcapsule shell materials are further described and exemplified U.S. Pat. No. 3,870,542. Amongst others the microcapsules described herein can be applied by any suitable process such as padding, spraying or wiping, with the microcapsules dispersed in a liquid as set out herein.

Microencapsulated cannabidiol is commercially available. Preferably, the microencapsulated cannabidiol is provided in the form of the product R-Vital 385 Hemp CBD (Vitalhemp Vertriebs GmbH, Austria).

Preferably, the microcapsule is chemically bonded to the gauze or warp yarns and weft yarns of the invention by means of a reactive functional group on the outer surface of the shell of the microcapsule that reacts with a reactive functional group on the gauze or warp yarns and weft yarns of the invention. In the case of such a chemical bonding, the microcapsule is bound to the gauze, or warp yarns and weft yarns via a covalent bond. In this context, the microcapsule can be directly bound to the gauze, or warp yarns and weft yarns or via a cross-linking agent.

Preferably, the microencapsulated anti-microbial agent, such as the microencapsulated cannabidiol, is bound to the gauze or warp yarns and weft yarns of the invention using a cross-linking agent. Such a cross-linking agent can create a covalent bond between the above-described microcapsules and the gauze or warp yarns and weft yarns of the invention. Alternatively, the crosslinking agent can act as a bridging agent between the microencapsulated anti-microbial agent and the surface of the gauze or warp yarns and weft yarns of the invention. Preferably, the cross-linking agent is a silane-based cross-linking agent. Suitable cross-linking agents, such as silane-based cross-linking agents, are commercially available. In preferred embodiments of the invention, the cross-linking agent is the silane-based cross-linking agent MIKRACAT B (Golden Technology Ltda., Brazil).

Any suitable wetting agent may be used in the context of the invention. Preferably, the wetting agent is an organic surfactant. Preferably the wetting agent comprises a hydroxy group. More preferably, the wetting agent is LAVA Wet MDF (Dystar Pte Ltd., Singapore).

In preferred embodiments of the invention, the anti-microbial treatment comprises microencapsulated CBD, a silane-based cross-linking agent and a wetting agent. More preferably, the anti-microbial treatment comprises microencapsulated CBD, a silane-based cross-linking agent and a wetting agent that comprises a hydroxy group. In particular preferred embodiments, the anti-microbial treatment comprises R-Vital 385 Hemp CBD, MIKRACAT B and LAVA Wet MDF. In most preferred embodiments, the anti-microbial treatment comprises R-Vital 385 Hemp CBD, MIKRACAT B and LAVA Wet MDF, wherein the masses of the R-Vital 385 Hemp CBD, MIKRACAT B and LAVA Wet MDF in the anti-microbial treatment are in a ratio 10:1:1.

In preferred embodiments of the invention, treating the woven gauze, or warp yarns and weft yarns of the woven gauze with the antimicrobial treatment, provides the gauze or warp yarns and weft yarns of the woven gauze with an anti-microbial surface. In preferred embodiments of the invention, the woven gauze according to the invention comprises an anti-microbial surface.

In preferred embodiments of the invention, the gauze, or warp yarns and weft yarns of the woven gauze comprise an anti-microbial agent as disclosed herein. Preferably, the antimicrobial anti-microbial agent is bound to the surface of the woven gauze, or warp yarns and weft yarns of the woven gauze, wherein the antimicrobial agent is bound directly or via crosslinking agent to the surface of the gauze, or warp yarns and weft yarns.

In preferred embodiments of the invention, the woven gauze is bleached. In particular preferred embodiments, the woven gauze of the invention is bleached by washing the woven gauze with H₂O₂ and NaOH. Preferred embodiments of the invention relate to bleaching of a woven gauze or warp yarns and/or weft yarns of the woven gauze. Preferably, the term "bleaching" refers to the treatment of the woven gauze or warp yarns and weft yarns of the woven gauze with a bleaching agent. A bleaching agent bleaches a material, therefore it lightens or whitens a substrate through chemical reaction. In preferred embodiments, the bleaching agent also acts as disinfectant.

In preferred embodiments of the invention, the bleaching agent is a chlorine-based bleaching agent (such as chlorine, a hypochlorite, a N-chloro compound or a chlorine dioxide) or a peroxygen-based bleaching agent (such as a hydrogen peroxide or a perborate). Preferably, the bleaching agent is an enzyme or is created by an enzyme. Enzymes can be used for generation of a bleaching agent such as an oxidizing bleaching agent. Exemplary enzyme bleaching agents are glucose oxidase, chloroperoxidase, laccase or catalase. Any suitable bleaching agent may be used. Preferably, the bleaching agent is an oxidizing bleaching agent. Preferably, the bleaching agent is a reducing bleaching agent. Preferably, the bleaching agent is selected from the group of hypochlorite (ClO⁻), chlorite (ClO₂⁻) and hydrogen peroxide (H₂O₂). Preferably, the bleaching agent comprises H₂O₂. In particular preferred embodiments of the invention, the bleaching agent comprises H₂O₂ under basic conditions, for example wherein the bleaching agent comprises mixture of H₂O₂ and an alkali metal hydroxide. The alkali metal preferably is selected from the group of lithium, sodium, potassium. In particularly preferred embodiments of the invention, the bleaching agent comprises a mixture of H₂O₂ and sodium hydroxide.

The invention further relates to a method for producing a woven multilayer gauze according to the invention, comprising the steps of
a) bleaching the woven gauze or the warp yarns and weft yarns before weaving the gauze, and
b) treating the woven gauze or the warp yarns and weft yarns before weaving with an anti-microbial treatment.

In this context, treating the woven gauze or the warp yarns and weft yarns before weaving with an anti-microbial treatment preferably refers to bringing into contact the woven gauze or the warp yarns and weft yarns before weaving with an anti-microbial treatment as disclosed herein.

In preferred embodiments of the invention the bleaching refers to treating the woven gauze or the warp yarns and weft yarns before weaving the gauze with a bleaching agent with a bleaching agent as disclosed herein. In particular preferred embodiments, the bleaching is conducted under rotation.

In preferred embodiments of the invention, the treating the woven gauze or the warp yarns and weft yarns before weaving the gauze with the bleaching agent is conducted for 1 h to 40 h, more preferably for 5 h - 30 h, more preferably for 10 h - 30 h, more preferably for 15 h - 25 h, most preferably for around 20 h.

In preferred embodiments, the treating the woven gauze or the warp yarns and weft yarns before weaving the gauze with a bleaching agent is conducted at an elevated temperature, in particular at 40 - 100 °C, more preferably at 60 - 100 °C, more preferably at 80 - 100 °C, most preferably at around 100 °C.

In preferred embodiments, the bleaching is followed by a step of drying the woven gauze or the warp yarns and weft yarns. Preferably said drying the woven gauze or the warp yarns and weft yarns is conducted at 70 - 160 °C, more preferably at 100 - 160 °C, more preferably at 120 - 160 °C, most preferably at around 140 °C.

In particularly preferred embodiments, the bleaching comprises treating the woven gauze or the warp yarns and weft yarns before weaving the gauze with a bleaching agent as described herein for 15 h - 25 h at a temperature of 80 - 100 °C, followed by drying at 120 - 160 °C. In particularly preferred embodiments, the bleaching comprises treating the woven gauze or the warp yarns and weft yarns before weaving the gauze with a bleaching agent as described herein for around 20 h at a temperature of around 100 °C, followed by drying at around 140 °C.

The present invention furthermore relates to an anti-microbial woven multilayer gauze that comprises an antimicrobial agent. Preferably, said woven multilayer gauze is a gauze according to any embodiment herein. Preferably, the anti-microbial woven multilayer gauze comprises an antimicrobial agent as disclosed herein. In preferred embodiments of the invention, the anti-microbial woven multilayer gauze of the invention comprises an anti-microbial surface, wherein the antimicrobial agent is bound directly or via crosslinking agent to the surface of the anti-microbial woven multilayer gauze.

Preferably, the woven gauze is designed as described above and below.

Preferred embodiments are given in the independent claims.

Further aspects, properties and features of the invention will become apparent and more appreciated from the following description of exemplary embodiments, taken in conjunction with the accompanying drawings, in which:
- Figure 1: perspective view on a woven multilayer gauze with multilayer section having three individual layers and one connecting section;
- Figure 2: an exemplary weave pattern of a woven multilayer gauze with the weave structure of a part of Figure 1;
- Figure 3: an exemplary weave pattern of a woven multilayer gauze with a multilayer section having three individual layers;
- Figure 4: an exemplary weave pattern showing a woven multilayer gauze with a multilayer section having five individual layers;
- Figure 5: a schematic top view on an inventive woven multilayer gauze;
- Figure 6: a schematic side view on a section of the woven multilayer gauze shown in Figure 1.

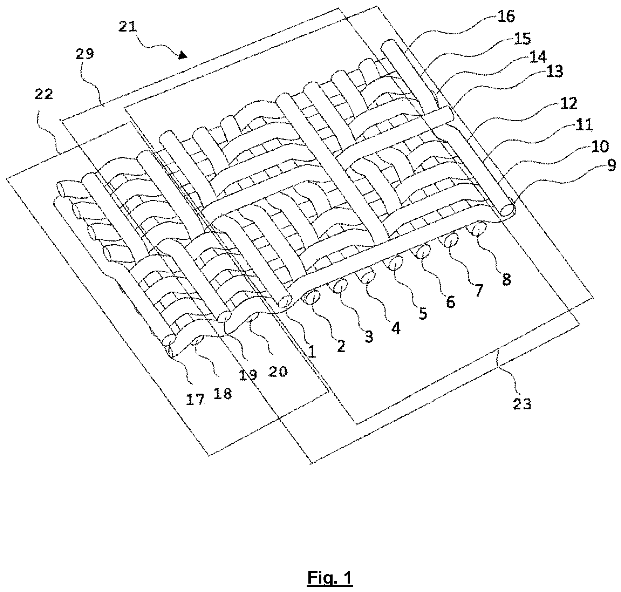

Figure 1 shows a woven multilayer gauze 21 comprising a multilayer section 23 with four individual layers, wherein the multilayer section 23 is bordered from the left side by a connecting section 22 in which yarns from each individual layer merge into a common layer.

The warp yarns are referenced with reference sign 1, 2, 3, 4, 5, 6, 7, 8, 17, 18, 19 and 20. The weft yarns are referenced with reference signs 9, 10, 11, 12, 13, 14, 15 and 16. The multilayer section 23 comprises four individual layers. The four individual layers will be referred to from the top to the bottom as first layer, second layer, third layer and fourth layer. The first layer is woven from warp yarns 1 and 5 and weft yarns 9 and 13. As can be seen, warp yarns 1 and 5 and weft yarns 9 and 13 form a plain weave extending above all warp yarns and weft yarns of the second, third and fourth individual layer of the multilayer section 23. The second layer is woven from warp yarns 2 and 6 and weft yarns 10 and 14 in a plain weave. As can be seen, the second individual layer extends below all warp and weft yarns of the first layer and above all warp and weft yarns of the third and fourth layer.

The third layer comprises warp yarns 3 and 7 and weft yarns 11 and 15. As can be seen, the third layer is woven as a plain weave and extends below all warp and weft yarns of the first and second layer and above all warp and weft yarns of the fourth layer. The fourth layer is woven from warp yarns 4 and 8 and weft yarns 12 and 16 in a plain weave and extends below all warp and weft yarns of the first, second and third layer.

The connecting section 22 shown in Figure 1 is a weft connecting section, in which the weft yarns 9, 10, 11, 12, 13, 14, 15 and 16 from the four layers of the multilayer section 23 merge into a common layer in which they are interwoven in a 1/1 weave with warp yarns 17, 18, 19 and 20, which can also be referred to as connecting warp yarns 17, 18, 19 and 20.

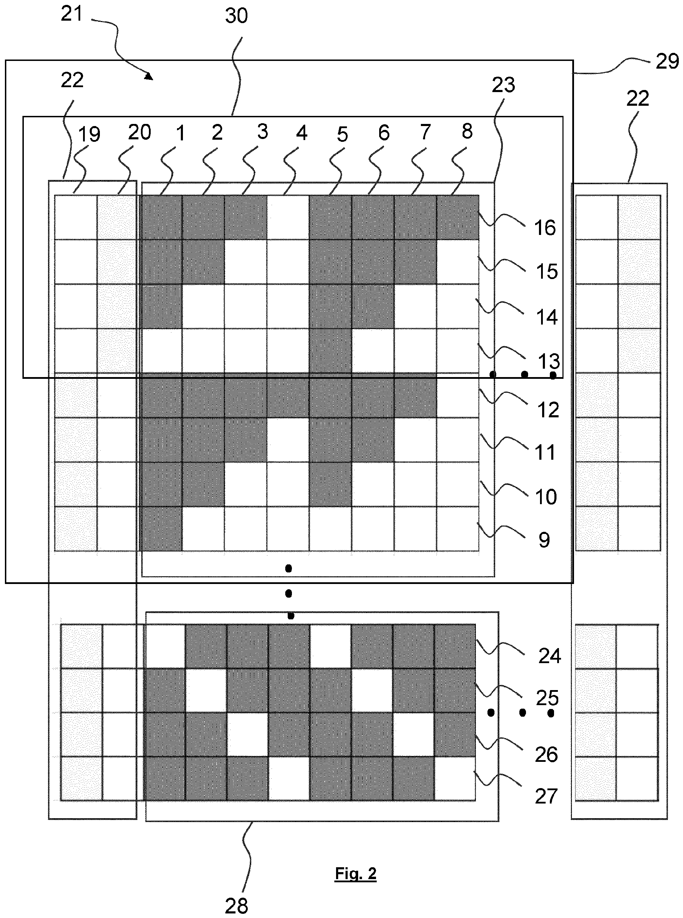

In Figure 2, the weave structure of the part of the multilayer gauze 21 in Figure 1 being bordered by the frame 29 is shown. Respectively, the warp yarns and the weft yarns are indicated with the same reference numbers. As indicated by the black dots, the multilayer section 23 extends further in warp and weft direction to provide a larger multilayer section depending on the length and width of the gauze bandage required. To provide a material gauze within the meaning of this invention, the multilayer section 23 can be bordered from both sides in weft direction by a connecting section 22 in the form of a weft connecting section as indicated in Figure 2. In the weft connecting sections 22 of Figures 1 and 2, each weft yarn 9, 10, 11, 12, 13, 14, 15 and 16 of each individual layer extends first over a single warp yarn 17, 18, 19 and 20 and subsequently under s single warp yarn, or vice versa. Further, in the weft connecting section 22, the warp yarns 17, 18, 19 and 20 extend over four weft yarns and subsequently under four weft yarns, or vice versa. In particular, the over portions of the warp yarns 17, 18, 19 and 20 build a diagonal pattern.

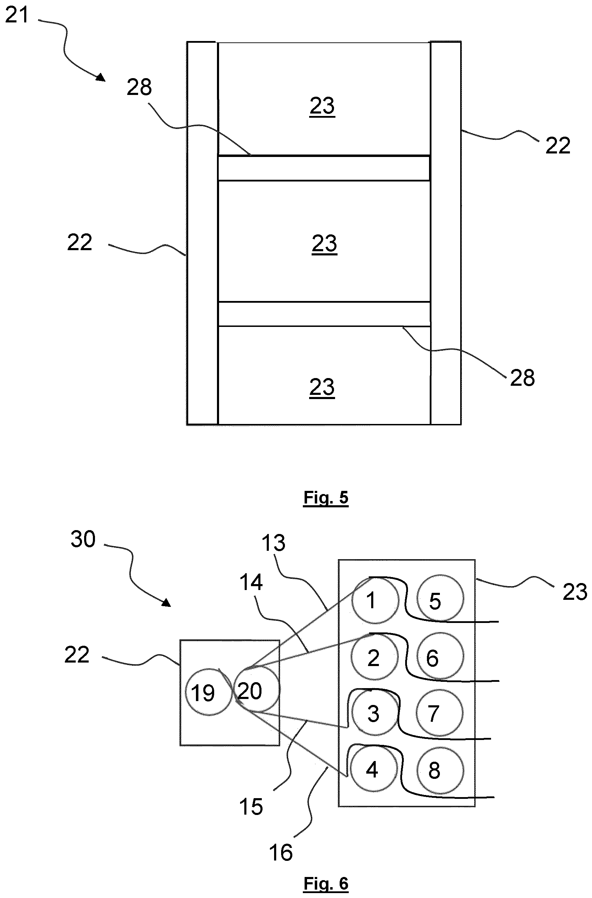

Figure 6 schematically shows a side view into a woven multilayer gauze 21 within the section 30 of Figure 2. As can be seen, the weft yarns 13, 14, 15 and 16 merge from the multilayer section 23, where they form plain weaves with their warp yarns 1, 5, 2, 6, 3, 7, 4 and 8 into the connecting section 22 where they form a common layer with their connecting warp yarns 19 and 20.

As indicated at the bottom of Figure 2, the multilayer section 23 can also be bordered in warp direction by a warp connecting section 28. The warp connecting section 28 of Figure 2 comprises for each individual layer of the multilayer section weft yarns extending over all warp yarns merging from that individual layer into the warp connecting section 28 and under all warp yarns merging from the other individual layers into the warp connecting section (for the purpose of illustration, in Figure 2, only one weft yarn is shown per individual layer. In detail, weft yarn 24 of the warp connecting section 28 extends over the warp yarns of the first layer, namely the warp yarns 1 and 5, but under the warp yarns of the second, third and fourth individual layer, namely warp yarns 2, 3, 4, 6, 7 and 8. Weft yarn 25 extends over the warp yarns of the second layer, namely over warp yarns 2 and 6, but under the warp yarns of the first, third and fourth individual layer, namely warp yarns 1, 3, 4, 5, 7 and 8. Weft yarn 26 extends over all warp yarns of the third individual layer, namely over warp yarns 3 and 7, but under the warp yarns of the first, second, fourth individual layer, namely warp yearns 1, 2, 4, 5 ,6 and 8. Weft yarn 27 extends over the warp yarns of the fourth layer, namely warp yarns 4 and 8, but under all warp yarns of the first, second and third layer, namely warp yarns 1, 2, 3, 5, 6 and 7. Thereby, the warp connecting section merges the warp yarns of the four individual layers of the multilayer section 23 into a common layer 28.

Figure 5 shows an embodiment of a woven multilayer gauze 21 with a plurality of multilayer sections 23 arranged behind each other in warp direction and being spaced from each other by warp connecting sections 28 and in addition being bordered at both sides in weft direction from weft connecting sections 22.

Each warp yarn 1, 2, 3, 4, 5, 6, 7 and 8 in the warp connecting section extends under one single weft yarn and subsequently over three weft yarns, namely the number of individual layers of the multilayer section 23 minus one. Thereby, the under portions of the weft yarns in the warp connecting section 28 build a diagonal pattern.

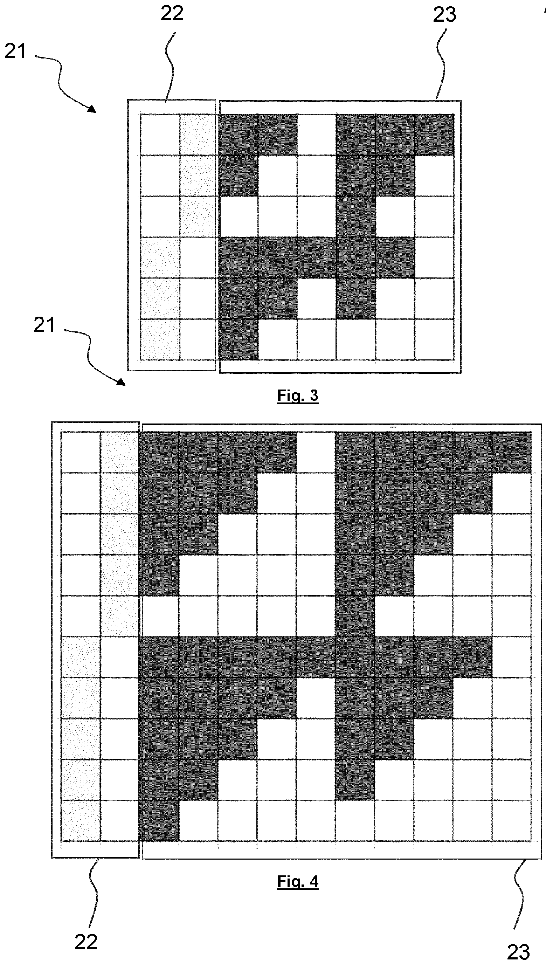

Figure 3 illustrates an embodiment of a woven multilayer gauze 21 similar to that of Figure 2, but with a multilayer section 23 having only three individual layers, being woven in a plain weave instead of four individual layers. Figure 4 shows an embodiment of a woven multilayer gauze 21 similar to that of Figure 2 with, however, five individual layers in the multilayer section 23 instead of four.

The features disclosed in the above description, the figures and the claims may be significant for the realization of the invention in its different embodiments individually as in any combination.

### Reference sings:

- 1, 2, 3, 4, 5, 6, 7, 8: warp yarns of multilayer section
- 9, 10, 11, 12, 13, 14, 15, 16: weft yarns of multilayer section
- 17, 18, 19, 20: warp yarn of weft connecting section
- 24, 25, 26, 27: weft yarns of warp connecting section
- 21: multilayer gauze
- 22: weft connecting section
- 23: multilayer section
- 28: warp connecting section

## Claims

1. A woven multilayer gauze (21), comprising at least one multilayer section (23) with at least three individual layers, wherein the at least one multilayer section (23) is bordered from at least two sides by connecting sections (22, 28) in which yarns from each individual layer merge into a common layer.

2. The woven multilayer gauze (21) according to claim 1, wherein the at least one multilayer section (23) is bordered in weft direction on both sides by weft connecting sections (22) in which the weft yarns from each layer of the multilayer section merge into a common layer.

3. The woven multilayer gauze (21) according to claim 2, wherein the warp yarn density in the weft connecting (22) sections is maximally as high as, in particular lower than, the warp density of the multilayer section (23), preferably is between 20 % and 80 %, more preferably between 30 % and 70 %, most preferably between 40 % and 60 %, of the warp density of the multilayer section.

4. The woven multilayer gauze (21) according to one of the claims 1 to 3, wherein at least one, preferably each, warp yarn in the common layer of at least one, preferably each, of the weft connecting sections (22) extends over exactly as much weft yarns as the number of individual layers in the multilayer section (23) and subsequently under exactly as much weft yarns as the number of individual layers of the multilayer section (23).

5. The woven multilayer gauze (21) according to one of the claims 1 to 4, wherein the at least one multilayer section (23) is bordered in warp direction on both sides by warp connecting sections (28) in which the warp yarns from each layer of the multilayer section (23) merge into a common layer.

6. The woven multilayer gauze (21) according to claim 5, wherein the weft yarn density in the warp connecting sections (28) is maximally as high as, in particular lower than, the weft density of the multilayer section (23), preferably is between 20 % and 80 %, more preferably between 30 % and 70 %, most preferably between 40 % and 60 %, of the weft density of the multilayer section (23).

7. The woven multilayer gauze (21) according to one of the claims 5 to 6, wherein at least one, preferably each of, the warp connecting sections (28) comprises for each individual layer of the multilayer section (23) at least one weft yarn extending over all warp yarns merging from that individual layer into the warp connecting section (28) and under all warp yarns merging from the other individual layers into the warp connecting (28) section.

8. The woven multilayer gauze (21) according to one of the claims 1 to 7, comprising at least two multilayer sections (23) with at least three individual layers, wherein each multilayer section (23) is bordered from at least two sides by connecting sections (22, 28) in which yarns from each individual layer merge into a common layer, wherein the two multilayer sections (23) are arranged behind each other in warp or weft direction and are spaced from each other by one of the connecting sections (22, 28).

9. The woven multilayer gauze (21) according to one of the claims 1 to 9 being wrapped in circumferential direction around the longitudinal axis of a roll, preferably wherein the woven gauze has a length in warp direction of at least 5 meter, preferably at least 10 meter or 20 meter, and a width in weft direction of at least 0,5 meter, preferably at least 1 meter or at least 2 meter.

10. The woven multilayer gauze (21) according to claim 9, wherein the at least one multilayer section (23) is bordered in circumferential direction from both sides by two connecting sections (22, 28) in which yarns, preferably warp yarns, from each individual layer merge into a common layer.

11. The woven multilayer gauze (21) according to claim 9 or 10, wherein the at least one multilayer section (23) is bordered along the longitudinal direction from both sides by two connecting sections (22, 28) in which yarns, preferably weft yarns, from each individual layer merge into a common layer.

12. The woven multilayer gauze (21) according to one of the preceding claims 1 to 11, wherein the woven gauze is enclosed by a cover, in particular a removable plastic cover.

13. The woven multilayer gauze (21) according to one of the claims 1 to 12,
wherein the woven gauze is bleached, in particular by treatment with H₂O₂ under basic conditions; and/or
wherein the woven gauze is treated with an anti-microbial treatment, in particular wherein the antimicrobial treatment comprises cannabidiol (CBD).

14. An anti-microbial woven multilayer gauze (21), wherein the anti-microbial woven multilayer gauze comprises an anti-microbial agent, preferably wherein the anti-microbial woven multilayer gauze is the anti-microbial woven multilayer gauze of any one of claims 1 to 14.

15. Method for producing a woven multilayer gauze (21) according to one of the preceding claims, comprising the steps of
a) bleaching the woven gauze or the warp yarns and weft yarns before weaving the gauze, preferably by treating the woven gauze or the warp yarns and weft yarns with H₂O₂ under basic conditions, and
b) treating the woven gauze or the warp yarns and weft yarns before weaving with an anti-microbial treatment, preferably wherein the anti-microbial treatment comprises an anti-microbial agent, a cross-linking agent, and a wetting agent.
